(19) Europäisches Patentamt

European Patent Office

Office européen des brevets

(11) EP 0 671 144 B1

(12) **EUROPEAN PATENT SPECIFICATION**

(45) Date of publication and mention
of the grant of the patent:
**24.02.1999 Bulletin 1999/08**

(51) Int. Cl.$^6$: **A61B 5/024**, A61B 5/022

(21) Application number: **94103632.9**

(22) Date of filing: **09.03.1994**

(54) **Pulse wave detecting apparatus**

Puls-Wellen-Messgerät

Appareil pour détecter des ondes pulsées

(84) Designated Contracting States:
**DE FR GB**

(43) Date of publication of application:
**13.09.1995 Bulletin 1995/37**

(73) Proprietor: **COLIN CORPORATION
Komaki-shi Aichi 485 (JP)**

(72) Inventors:
• **Takaya, Masami
Komaki-shi, Aixchi-ken (JP)**
• **Nishibayashi, Hideo
Komaki-shi, Aixchi-ken (JP)**

(74) Representative:
**Winter, Brandl, Fürniss, Hübner, Röss,
Kaiser, Polte, Kindermann
Partnerschaft
Patent- und Rechtsanwaltskanzlei
Alois-Steinecker-Strasse 22
85354 Freising (DE)**

(56) References cited:
**EP-A- 0 401 382       EP-A- 0 452 578
WO-A-93/15653       GB-A- 2 257 529
US-A- 5 005 581**

## Description

## BACKGROUND OF THE INVENTION

Field of the Invention

The present invention relates to a pulse wave detecting apparatus according of the preamble of claim 1 which presses a pulse wave sensor against an arterial vessel of a living body or subject via body surface above the artery and detects a pressure pulse wave produced from the artery.

Related Art Statement

There is known a pulse wave detecting device including (a) a pulse wave sensor having a press surface and including a plurality of pressure sensing (PS) elements provided in one or more arrays in the press surface, the pulse wave sensor being adapted to be pressed against an arterial vessel of a living subject via a body surface of the subject such that a direction of the array or arrays of PS elements intersects a direction of extension of the artery, so that each of the pressure sensing elements detects a pressure pulse wave produced from the artery and generates a pulse wave signal representing the detected pulse wave, (b) a pressing device which produces a pressing force to press the press surface of the pulse wave sensor against the artery via the body surface, and (c) regulating means for continuously changing the pressing force of the pressing device applied to the pulse wave sensor, determining an optimum value of the pressing force based on one or more of the pulse wave signals generated from the PS elements, and holding the pressing force of the pressing device at the thus determined optimum value. The prior apparatus reads in the pulse wave signal or signals supplied from one or more of the PS elements pressed with the optimum pressing force, and obtains the pressure pulse wave of the subject based on the pulse wave signal or signals. The prior apparatus is disclosed in, for example, Japanese Patent Application laid open for inspection purpose under Publication No. 1(1989)-285244.

However, in the prior pulse wave detecting device, the condition of pressing of the pulse wave sensor against the body surface (e.g., manner of contact of the former with the latter) may change due to, for example, motion of the subject (e.g., motion of his or her wrist on which the sensor is being worn). In this event, the reading or detected magnitude of the pulse wave is adversely affected. The detected pulse wave may contain both change due to natural change of blood pressure of the subject and change due to artificial change of the pressing condition of pulse wave sensor. Thus, the accuracy of detection of the prior device is not satisfactory.

From document EP-A-0401382 a pulse wave detecting apparatus including a pulse wave sensor, a pressing device including a diaphragm, a pressure chamber, a pressure regulator wave and a pump, and a regulating means in form of a CPU is disclosed. This disclosed apparatus determines a variation of the minimum values of the pulse waves obtained from the optimum pressure sensing element while the pressing force, i.e. pressure PM in pressure chamber 38, applied to the optimum pressure sensing element is changed before an optimum pressing force is determined and the pressing force of the pressing device is held at the optimum value.

From document US-A-5005581 an apparatus for detecting motion artefacts are disclosed. This motion artefacts are based on a change of the past pressure outputs. The apparatus includes the steps of monitoring the amplitude of a pulse wave form from a first pulse to a next successive pulse and determining if the output signal changes by more than a predetermined percentage, thus indicating a motion condition.

## SUMMARY OF THE INVENTION

It is therefore an object of the present invention to provide a pulse wave detecting apparatus which continuously detects a pressure pulse wave of a living subject with high accuracy.

This object is solved by the features of claim 1.

The Applicants have intensively studied for achieving the above object, and have found that the variation (i.e., distribution or pattern) of the respective minimum magnitudes of the pulse waves detected through the pressure sensing elements with respect to the array of pressure sensing elements, with the pulse wave sensor being pressed with the optimum pressing force, is intimately related to the condition of pressing of the pulse wave sensor against the body surface of the subject. The present invention has been developed based on this discovery.

The above object has been achieved by the present invention, which provides a pulse wave detecting apparatus for detecting a pulse wave from a living subject, the pulse wave comprising a plurality of pulses produced from an arterial vessel of the subject in synchronism with heartbeat of the subject, comprising (a) a pulse wave sensor having a press surface and including at least one array of pressure sensing elements provided in the press surface, the press surface of the pulse wave sensor being adapted to be pressed against the arterial vessel of the living subject via a body surface of the subject such that a direction of the array of pressure sensing elements intersects a direction of extension of the arterial vessel, so that each of the pressure sensing elements detects the pulse wave produced from the arterial vessel and generates a pulse wave signal representing the detected pulse wave, (b) a pressing device which produces a pressing force to press the press surface of the pulse wave sensor against the arterial vessel via the body surface, (c) regulating means for changing the pressing force of the pressing device applied to the pulse wave sensor, determining an optimum value of the pressing force based on at least one of the pulse wave signals generated from the pressure sensing elements, and holding the pressing force of the pressing device at the thus determined optimum value, (d) minimum magnitude distribution determining means for determining a minimum magnitude of at least one pulse of each of the pulse waves represented by the respective pulse wave signals from the pressure sensing elements, the minimum magnitude distribution determining means iteratively determining a distribution of the respective minimum magnitudes of the pulse waves with respect to the array of pressure sensing elements after the pressing force of the pressing device is held at the optimum value, and (e) judging means for judging whether a pressing condition of the pulse wave sensor on the body surface is stable, based on change of the minimum magnitude distributions determined by the minimum magnitude distribution determining means.

In the pulse wave detecting apparatus constructed as described above, the minimum magnitude determining means determines iteratively, determines a distribution or a variation of the respective minimum magnitudes of the pulse waves with respect to the array of pressure sensing elements after the pressing force of the pressing device is held at the optimum value, and the judging means judges whether a pressing condition of the pulse wave sensor on the body surface is stable, based on change of the minimum magnitude distributions determined by the minimum magnitude distribution determining means. When the judging mans provides a negative judgment that the pressing condition of the pulse wave sensor on the body surface is not stable, i.e., has changed, the regulating means may be operated for updating the optimum pressing force of the pressing device, thereby changing the pressing condition of the pulse wave sensor. Alternatively, the present apparatus may further comprise an informing device which informs an operator or user of the negative judgment, so that the operator or user can recognize that the pressing condition of the pulse wave sensor has changed. Thus, the accuracy of detection of the present apparatus is improved.

## BRIEF DESCRIPTION OF THE DRAWINGS

The above and optional objects, features, and advantages of the present invention will be better understood by reading the following detailed description of the preferred embodiments of the invention when considered in conjunction with the accompanying drawings in which:

Fig. 1 is a diagrammatic view of a pulse wave detecting apparatus embodying the present invention;

Fig. 2 is an enlarged view of a pulse wave sensor incorporated in the apparatus of Fig. 1, as seen by a person facing the press surface of the sensor in which surface an array of pressure sensing elements are provided;

Figs. 3(a) and 3(b) are flow charts representing respective parts of the control program used by the apparatus of Fig. 1;

Figs. 4(a) and 4(b) are flow charts representing other parts of the control program used by the apparatus of Fig. 1;

Fig. 5 is a view of a reference minimum tonogram curve, $MTC_s$, and a subsequent minimum tonogram curve, MTC, each determined at Step S9 of the flow chart of Fig. 3;

Fig. 6 is a view of (a) a reference curve $MTC_s$ and (b) a subsequent curve MTC translated so that the two curves $MTC_s$, MTC take an identical value with respect to an optimum pressure sensing element of the pulse wave sensor of Fig. 2;

Figs. 7(a) to 7(j) are views of various patterns of change of a subsequent curve MTC from a reference curve $MTC_s$, each pattern belonging to a first group of patterns, I, which indicate that the pressing condition of the pulse wave sensor is stable;

Figs. 8(a) to 8(l) are views of various patterns of change of a subsequent curve MTC from a reference curve $MTC_s$, each pattern belonging to a second group of patterns, II, which indicate that the pressing condition of the pulse wave sensor is not stable; and

Figs. 9(a) to 9(d) are views of various patterns of change of a subsequent curve MTC from a reference curve $MTC_s$, each pattern belonging to a third group of patterns, II, which indicate that the pressing condition of the pulse wave sensor is not stable.

In the above figures the minimum magnitude of the pulse waves is called "lower peak magnitude".

## DETAILED DESCRIPTION OF THE PREFERRED EMBODIMENTS

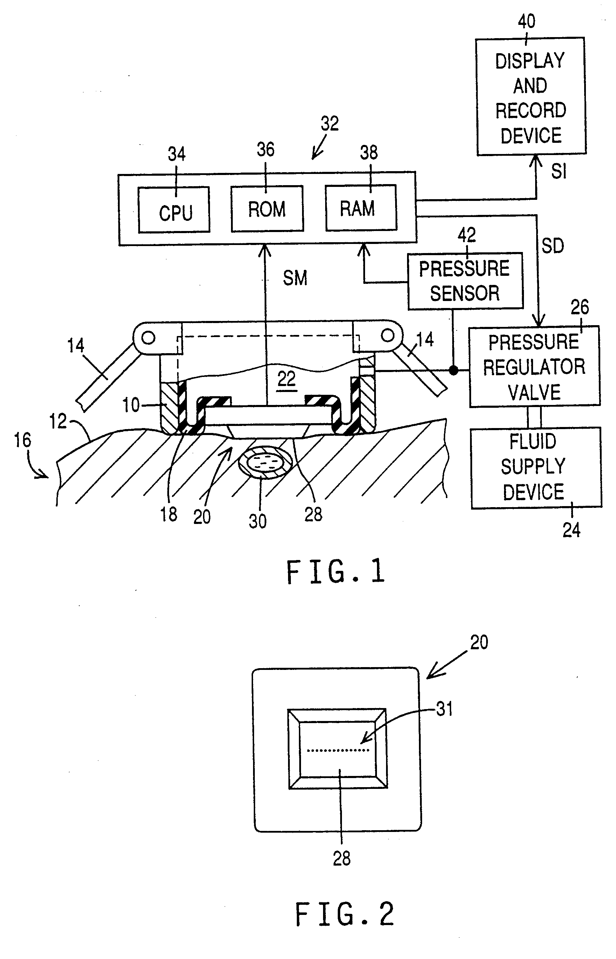

Referring first to Fig. 1, there is shown a pulse wave detecting apparatus embodying the present invention. In the figure, reference numeral 10 designates a container-like housing. The housing 10 has a wall-free end closed by a pulse wave (PW) sensor 20 and a diaphragm 18. The present apparatus is detachably set on a wrist 16 of a living subject with the help of setting bands 14, 14, such that the wall-free end of the housing 10 is held in contact with body surface 12 of the subject 16. The diaphragm 18 is flexible enough to permit the PW sensor 20 to displace relative to the housing 10 and advance out of the wall-free end of the housing 10. The housing 10, diaphragm 18, and PW sensor 20 cooperate with each other to define a pressure chamber 22, which is supplied with pressurized fluid such as pressurized air from a fluid supply device 24 via a pressure regulator valve 26. A pressure sensor 42 detects the fluid pressure in the pressure chamber 22 (hereinafter, referred to as the "chamber pressure P"), and generates an electric signal representing the detected chamber pressure P. The PW sensor 20 is pressed on the body surface 12 with a pressing force corresponding to the chamber pressure P. In the present embodiment, the housing 10, diaphragm 18, fluid supply 24, and pressure regulator 26 cooperate with each other to serve as a pressing device for pressing the PW sensor 20 against the body surface 12.

As shown in Fig. 2, the PW sensor 20 has a press surface 28 defined by a semiconductor chip of, for example, monocrystalline silicon. A single array of pressure sensing (PS) elements 31 (e.g., thirty PS diodes) are provided straightly in the press surface 28. However, the PS elements 31 may be arranged in two or more arrays. The PW sensor 20 is pressed against a radial artery 30 via the body surface 12 such that the direction of the array of PS elements 31 generally perpendicularly intersects the direction of extension of the radial artery 30, so that each of the PS elements 31 detects an oscillatory pressure wave or pressure pulse wave that is produced from the radial artery 30 and is propagated to the body surface 12. A pressure pulse wave contains a plurality of pulses produced from an arterial vessel of a living subject in synchronism with heartbeat of the subject. The PS elements 31 are provided equidistantly from each other in the press surface 28, and the distance between the PS elements 31 is preselected at a sufficiently small value which enables a sufficiently great number of PS elements 31 to ride directly above the radial artery 30. Additionally, the overall length of the array of PS elements 31 is pre-selected at a greater value than the lumen or diameter of the radial artery 30. Each of the PS elements 31 generates an electric signal, i.e., pulse wave signal, SM, representing the pressure pulse wave detected thereby from the radial artery 30, and the pulse wave signals SM generated from all the PS elements 31 are supplied to a control device 32. The control device 32 also receives from the pressure sensor 42 the pressure signal representing the chamber pressure P.

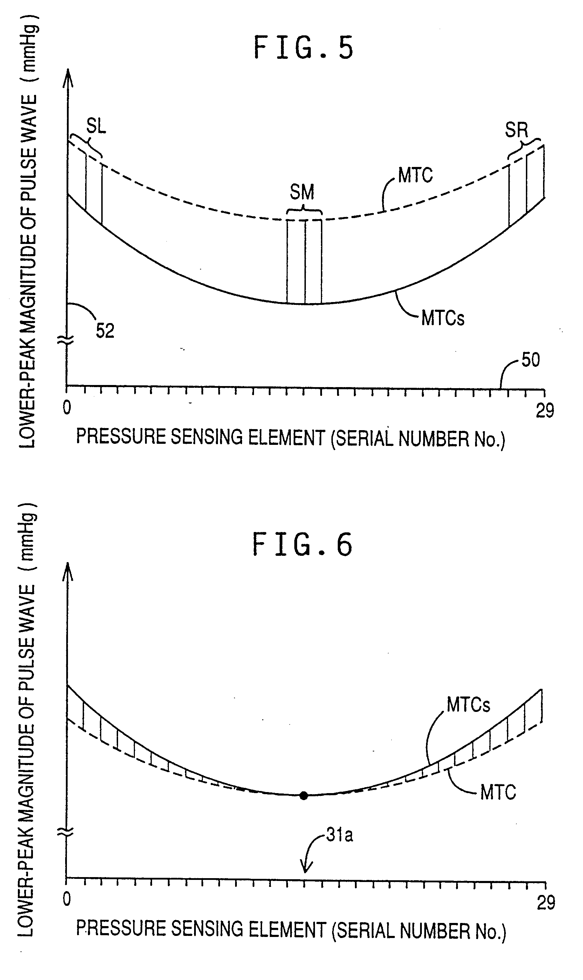

The control device 32 is essentially constituted by a microcomputer including a central processing unit (CPU) 34, a read only memory (ROM) 36, and a random access memory (RAM). The CPU 34 processes input signals according to control programs pre-stored in the ROM 36 by utilizing temporary-data-storage function of the RAM 38. Specifically, according to well-known control programs, the CPU 34 determines an optimum pressing force (in the present embodiment, optimum chamber pressure, Pa) to be applied to the PW sensor 20, and selects an optimum PS element 31a from the array of PS elements 31, based on the pulse wave signals SM supplied from the PS elements 31 while the chamber pressure P is changed. The CPU 34 supplies control signal, SD, to the pressure regulator 26 to hold the chamber pressure P at the thus determined optimum value Pa. After the chamber pressure P is held at the optimum value Pa, the CPU 34 reads in, and stores in the RAM 38, the pressure pulse wave in the form of the pulse wave signal SM supplied from the optimum PS element 31a, and supplies control signal, SI, to a display and record device 40 to display and record the thus obtained pulse wave that contains heartbeat-synchronous pulses. Meanwhile, the CPU 34 determines a minimum magnitude of one or more pulses of the pulse wave represented by the pulse wave signal SM supplied from each of the PS elements 31. The CPU 34 determines a distribution, MTC (Fig. 5), of the minimum magnitudes of the pulse waves with respect to the array of PS elements 31, in each of periodic cycles after the chamber pressure P is held at the optimum value Pa. The CPU 34 judges whether or not the condition of pressing of the PW sensor 20 on the body surface 12 is stable, based on time-wise change of the pulse-wave minimum magnitude distribution determined in the periodic cycles. In the event that the CPU 34 judges that the pressing condition of the PW sensor 20 is not stable, i.e., has changed due to, e.g., motion of the wrist 16, the CPU 34 updates the optimum chamber pressure Pa and holds the chamber pressure P at the updated optimum pressure Pa. Thus, the present apparatus continues to press the PW sensor 20 against the body surface 12 under the stable condition, so that the optimum PS element 31a continues to detect the pressure pulse wave (i.e., pulse wave signal SM) with high accuracy. In the following description, the pulse-wave minimum magnitude distribution determined by the CPU 34 in each determination cycle is referred to as the "minimum tonogram curve MTC". In the present embodiment, the CPU 34 does not determine a "literal" curve MTC as illustrated in Fig. 5. However, as the number of the PS elements 31 employed increases, the pulse-wave minimum magnitude distribution determined approaches the curve MTC.

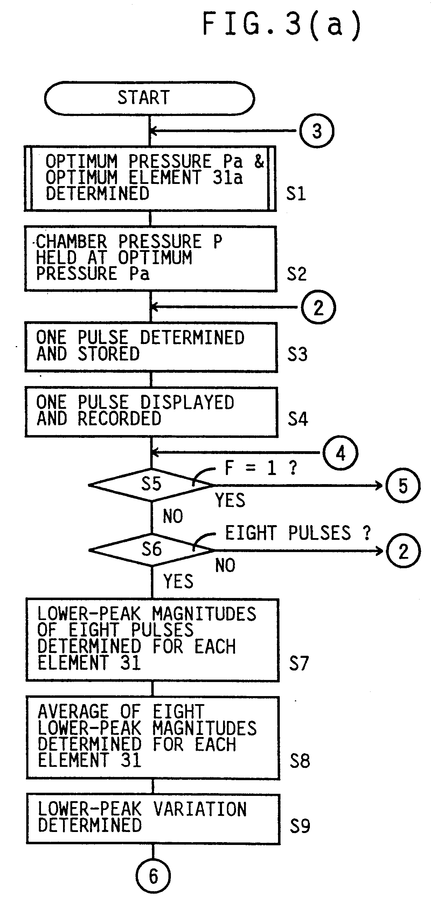

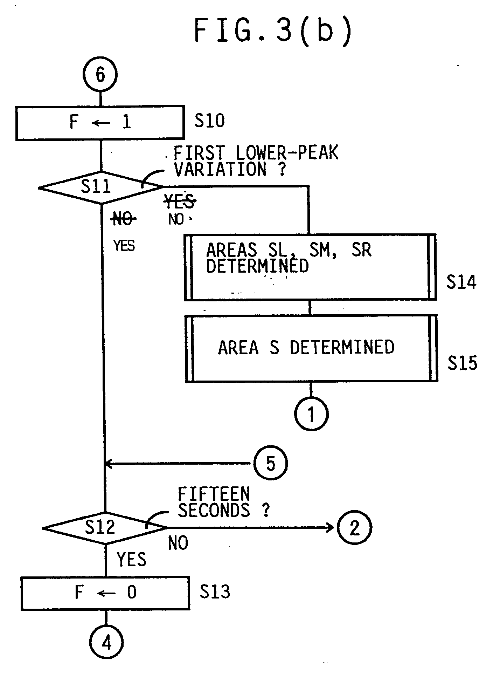

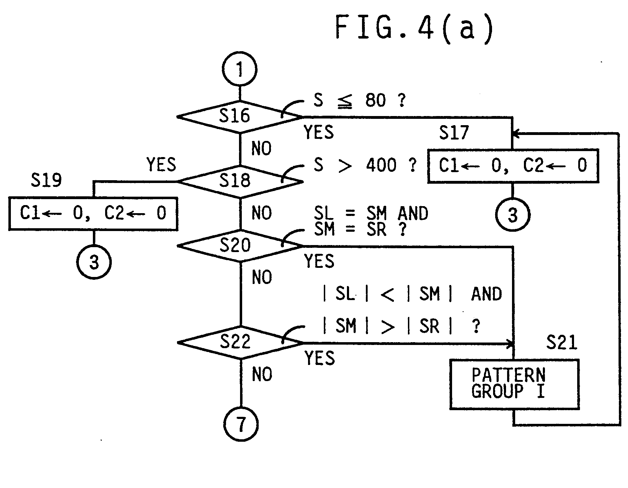

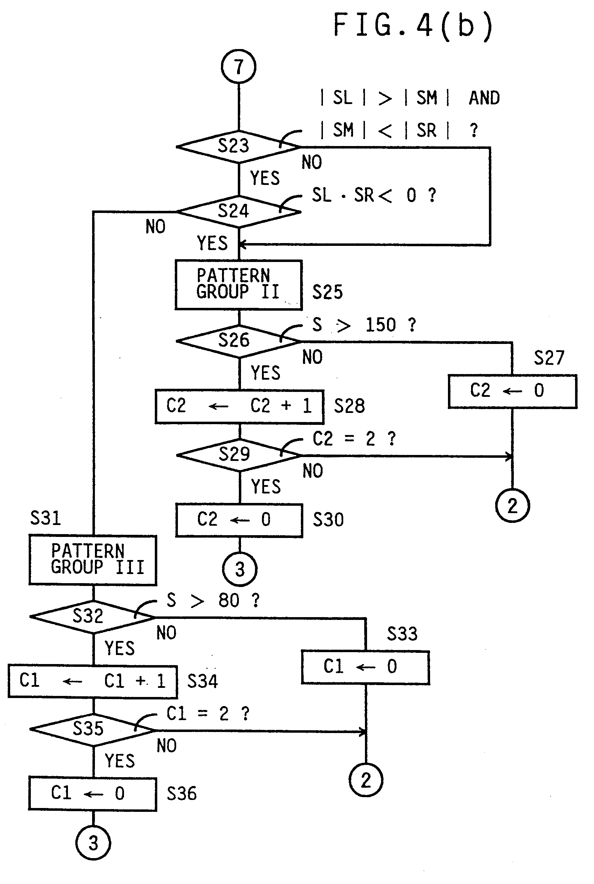

Hereinafter there will be described the operation of the pulse wave detecting apparatus constructed as described above, by reference to the flow charts of Figs. 3(a), 3(b), 4(a), and 4 (b).

Upon application of electric power to the present apparatus, initialization of the apparatus is carried out in a step (not shown) in which flag, F, and counters, $C_1$ and $C_2$, (described later) each are reset to zero. Subsequently, when a start and stop switch (not shown) is operated to its "ON" state, the control of the CPU 34 starts with Step S1 to operate the pressure regulator 26 so as to allow fluid to discharge from the pressure chamber 22 and subsequently supply the chamber 22 with the pressurized fluid fed from the supply device 24. Specifically, as the pressurized fluid is fed, the chamber pressure P is slowly and gradually increased up to a predetermined level, e.g., about 250 mmHg.

During this chamber pressure P increasing operation, the CPU 34 receives the pulse wave signal SM from each of the PS elements 31 and the pressure signal from the pressure sensor 42 which signal represents the chamber pressure P currently being increased. The CPU 34 determines the amplitude (i.e., difference between the upper-peak and lower-peak magnitudes or between the maximum and minimum magnitudes) of each pulse of the pulse wave signal SM from each of the PS elements 31, and selects as the optimum PS element 31a one of the PS elements 31 which has supplied a maximum pulse having a maximum amplitude. Additionally, the CPU 34 determines as the optimum pressure Pa the chamber pressure P at the time of detection (or reception) of the maximum pulse from the optimum PS element 31a.

Step S1 is followed by Step S2 to hold the chamber pressure P at the optimum pressure Pa determined at Step S1. In this situation, the wall of the radial artery 30 is partially flattened under the press surface 28 of the PW sensor 20, as shown in Fig. 1. In the present embodiment, the pressure sensor 42, Steps S1 and S2 of Fig. 3(a), and a portion of the control device 32 to carry out those steps cooperate with each other to serve as regulating means for regulating the pressing force of the pressing device 18, 22, 24, 26 so as to press the PW sensor 20 with the optimum pressing force Pa.

At the following step, Step S3, the CPU 34 reads in signal SM corresponding to one pulse, from each of the PS elements 31, and stores the one-pulse signals SM from all the PS elements 31 in an appropriate area of the RAM 38. Step S3 is followed by Step S4 to operate the display/record device 40 to display and record the one-pulse signal SM supplied from the optimum PS element 31a and stored in the RAM 38. At the following step, Step S5, the CPU 34 judges whether the content of flag F is 1 (i.e., F=1). Since flag F is reset to 0 (i.e., F=0) at the time of initialization, a negative judgment is made at Step S5 in the current control cycle. Therefore, the control of the CPU 34 proceeds with Step S6.

At Step S6, the CPU 34 judges whether the CPU 34 has read in a total of eight pulses from each PS element 31 after the chamber pressure P is held at the optimum pressure Pa which has been determined for the first time after the start/stop switch is operated to the "ON" state. So long as a negative judgment is made at Step S6, Steps S3 to S6 are repeated. Meanwhile, if a positive judgment is made at Step S6, the control of the CPU 34 goes to Step S7 to determine the minimum magnitude of each of the last eight pulses supplied from each PS element 31 and stored in the RAM 38. Step S7 is followed by Step S8 to determine an average of the thus determined eight minimum magnitudes with respect to each of the PS elements 31.

Subsequently, at Step S9, the CPU 34 determines a current minimum tonogram curve MTC as indicated in solid line in the two-dimensional coordinate system of Fig. 5. This coordinate system has an axis of abscissa 50 indicative of the serial number (No.) assigned to each PS element 31 of the PW sensor 20, and an axis of ordinate 52 indicative of the minimum magnitude of the pulse wave (i.e., pulse wave signal SM) in terms of mmHg. The current curve MTC is obtained by plotting in the graph a point representing the average minimum magnitude of each PS element 31, and connecting the plotted points with a line. Serial numbers (Nos.) are assigned to the respective PS elements 31 in the order of location thereof in the array provided in the press surface 28 of the PW sensor 20. Step S9 is followed by Step S10 to set flag F to F=1, indicating that a curve MTC has been determined at Step S9. Subsequently, at Step S11, the CPU 34 judges whether the curve MTC determined at Step S9 in the current control cycle is the first one determined after the chamber pressure P is last held at the optimum pressure Pa. Immediately after the beginning of operation of the apparatus, a positive judgment is made at Step S11, so that the control of the CPU 34 goes to Step S12. Hereinafter, the curve MTC determined immediately after the chamber pressure P is last held at the optimum pressure Pa is referred to as the "reference curve $MTC_s$".

At Step S12, the CPU 34 judges whether fifteen seconds have passed after a curve MTC (for the current control cycle, reference curve $MTC_s$) is determined at Step S9. If a negative judgment is made at Step S12, Steps S3-S5 and S12 are repeated to detect, store, display, and record respective pulses of the pulse wave from the optimum PS element 31a. Meanwhile, if a positive judgment is made at Step S12, the control of the CPU 34 goes to Step S14 to reset flag F to F=0, and subsequently the control of the CPU 34 returns to Step S5. Since flag F has just been reset to F=0 at Step S13, a negative judgment is made at Step S5. Since, at Step S6, the CPU 34 always obtains a positive result after having once obtained a positive judgment at this step, the control of the CPU 34 goes to Steps S7-S9 to determine a current minimum tonogram curve MTC as indicated in broken line in Fig. 5. Then, at Step S10, the CPU 34 sets flag F to F=1, and subsequently the control of the CPU 34 goes to Step S11. At this time, a negative judgment is made at Step S11, therefore the control of the CPU 34 goes to Step S14 and the following steps. Thus, after the determination of the reference curve MTCs, a current curve MTC is determined at regular intervals of 15 seconds.

At Step S14, the CPU 34 calculates a first-end area, SL, an optimum-portion area, SM, and a second-end area, SR, (Fig. 5) for the purpose of determining a pattern of change of the current curve MTC from the reference curve $MTC_s$. In the present embodiment, the first end area SL is calculated or approximated by summing the values obtained by subtracting the respective values on the reference curve $MTC_s$ from the corresponding values on the current curve MTC, with respect to each of the three PS elements 31 located at one (i.e.,

left-hand end in Fig. 5) of opposite ends of the array of PS elements 31 of the PW sensor 20. Similarly, the second-end area SR is approximated by summing the values obtained by subtracting the respective values on the reference curve $MTC_s$ from the corresponding values on the current curve MTC, with respect to each of the three PS elements 31 located at the other end (i.e., right-hand end in Fig. 5) of the array of PS elements 31. The optimum-portion area SM is approximated by summing the values obtained by subtracting the respective values on the reference curve $MTC_s$ from the corresponding values on the current curve MTC, with respect to each of the three PS elements 31 consisting of the optimum PS element 31a and two adjacent PS elements 31 located on both sides of the element 31a in the array of PS elements 31.

Step S14 is followed by Step S15 to calculate an overall amount of change, S, of the current curve MTC from the reference curve $MTC_s$, according to the following expression (1):

$$S = \sum_{i=0}^{29} |\{MTC(i) - diff\} - MTC_s(i)|$$

, where

i: the serial number (No.) assigned to each PS element 31;
MTC(i): the value on the curve MTC with respect to the element 31 numbered "i";
$MTC_s$(i): the value on the curve $MTC_s$ with respect to the element 31 numbered "i"; and
diff: the value obtained by subtracting the value on the curve $MTC_s$ from the corresponding value on the curve MTC with respect to the optimum PS element 31a.

The change amount S approximates the change area enveloped by (a) the reference curve $MTC_s$ and (b) the current curve MTC translated along the axis of ordinate 52 by subtracting the value, diff, from the respective values on the curve MTC with respect to all the PS elements 31a, as shown in Fig. 6. In the coordinate system of Fig. 6, the two curves $MTC_s$, MTC take an identical value with respect to the optimum PS element.

At the following step, S16, in Fig. 4 (a), the CPU 34 judges whether the change area S is not greater than a first reference value, e.g., 80. A positive judgment at Step S16 that the change area S is not greater than 80 indicates that the change of the pulse waves (or pulse wave signals SM) represented by the change area S resulted from natural change of blood pressure of the subject 16 and therefore that the pressing condition of the PW sensor 20 against the body surface 12 is stable. Thus, the control of the CPU 34 goes to Step S17 to reset first and second counters $C_1$, $C_2$ each to zero ($C_1$=0, $C_2$=0). Then, the control of the CPU 34 returns to Step S3 to continue to detect the pressure pulse wave through the optimum PS element 31a. On the other hand, if a negative judgment is made at Step S16, the control of the CPU 34 goes to Step S18 to judge whether the change area S is greater than a second reference value, e.g., 400, greater than the first reference value employed at Step S16. A positive judgement at Step S18 that the change area S is greater than 400 indicates that the change of the pressure pulse wave represented by the change area S did not result from artificial change of blood pressure of the subject and therefore that the pressing condition of the PW sensor 20 against the body surface 12 is not stable. Thus, the control of the CPU 34 goes to Step S19 to reset first and second counters $C_1$, $C_2$ to $C_1$=0 and, $C_2$=0, respectively. Then, the control of the CPU 34 returns to Step S1 to re-determine or update the optimum chamber pressure Pa, re-select or update the optimum PS element 31a, hold the chamber pressure P at the updated optimum pressure Pa, and resume detecting the pressure pulse wave of the subject through the updated optimum PS element 31a.

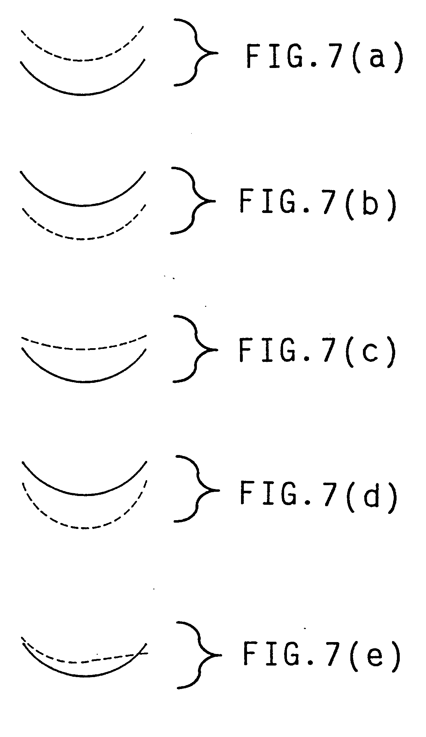

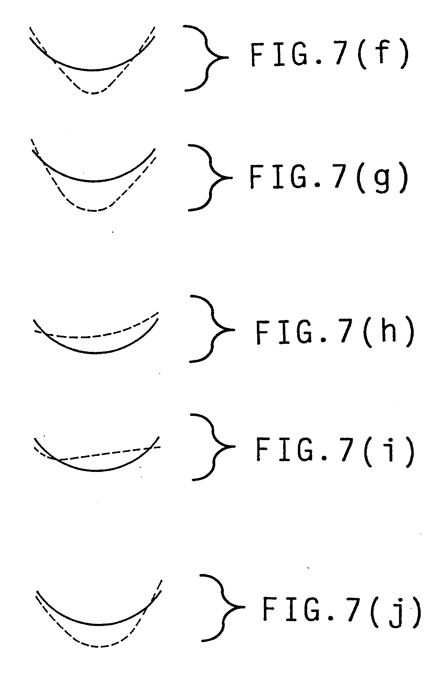

On the other hand, if a negative judgment is made at Step S18, i.e., if the change area S falls within the range, $80 < S \leq 400$, the control of the CPU 34 goes to Step S20 to judge whether the first-end area SL is equal to the optimum-portion area SM and simultaneously the optimum-portion area SM is equal to the second-end area SR. If a positive judgment is made at Step S20, the control goes to Step S21 to conclude that the pattern of change of the current curve MTC from the reference curve $MTC_s$ corresponds to a first group of patterns, I, more specifically, one of patterns shown in Figs. 7(a) and 7(b). This result indicates that the pressing condition of the PW sensor 20 on the body surface 12 is stable. Therefore, the control of the CPU 34 goes to Step S17 to reset counters $C_1$, $C_2$ to $C_1$=0 and $C_2$=0, respectively, and subsequently returns to Step S3 to continue to detect the pressure pulse wave of the subject 16.

On the other hand, if a negative judgment is made at Step S20, the control of the CPU 34 goes to Step S22 to judge whether the absolute value of the first-end area SL is smaller than the absolute value of the optimum-portion area SM and simultaneously the absolute value of the optimum-portion area SM is greater than the absolute value of the second-end area SR. If a positive judgment is made at Step S22, the control goes to Step S21 to conclude that the pattern of change of the current curve MTC from the reference curve $MTC_s$ corresponds to the first pattern I, more specifically, one of patterns shown in Figs. 7(c) to 7(j) wherein both of the end portions of the current curve MTC did not change from the corresponding portions of the reference curve $MTC_s$. This result indicates that the change of the pressure pulse wave represented by the change area S resulted from the change of blood pressure of the subject and that the pressing condition of the PW sensor 20 on the body surface 12 is stable. Therefore, the control of the CPU 34 goes to Step S17 to reset counters $C_1$, $C_2$ to $C_1=0$ and $C_2=0$, respectively, and subsequently returns to Step S3 to continue to detect the pressure pulse wave of the subject 16.

On the other hand, if a negative judgment is made at Step S22, the control of the CPU 34 goes to Step S23 to judge whether the absolute value of the first-end area SL is greater than the absolute value of the optimum-portion area SM and simultaneously the absolute value of the optimum-portion area SM is smaller than the absolute value of the second-end area SR. If a positive judgment is made at Step S23, the control goes to Step S24 to judge whether the product of the first-end area SL and the second-end area SR is negative, i.e., judge whether one of the two areas SL, SR is negative and the other area SL, SR is positive.

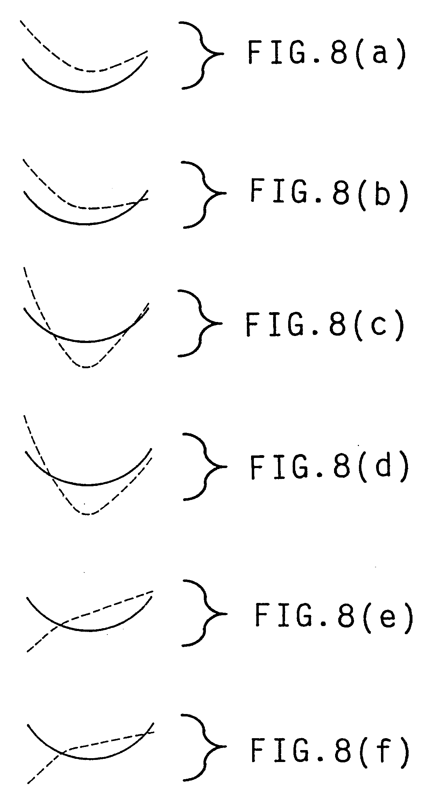

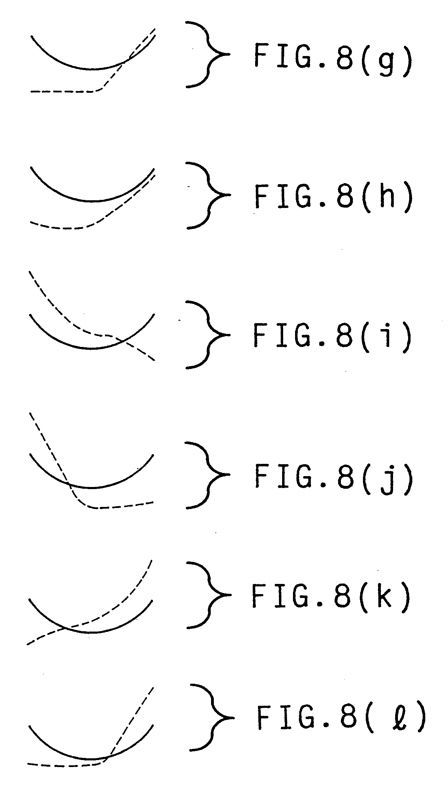

If a positive judgment is made at Step S24, the control goes to Step S25 to conclude that the pattern of change of the current curve MTC from the reference curve $MTC_s$ corresponds to a second group of patterns, II, more specifically, one of patterns shown in Figs. 8(i) to 8(l) wherein both of the end portions of the current curve MTC largely changed from the corresponding portions of the reference curve $MTC_s$. This result suggests that the change of the pressure pulse wave represented by the change area S did not result from the change of blood pressure of the subject and that the pressing condition of the PW sensor 20 is not stable, i.e., has changed. Subsequently, the control of the CPU 34 goes to Step S26 to judge whether the change area S Is greater than a third reference value, e.g., 150, greater than the first reference value used at Step S16 and smaller than the second reference value used at Step S18. A positive judgement at Step S26 that the change area S is greater than 150, indicates that the pressing condition of the PW sensor 20 against the body surface 12 has not changed, i.e., remains stable. Thus, the control of the CPU 34 goes to Step S27 to reset counter $C_2$ to $C_2=0$. Then, the control of the CPU 34 returns to Step S3 to continue to detect the pressure pulse wave of the subject 16.

On the other hand, if a positive judgment is made at Step S26, the control of the CPU 34 goes to Step S28 to add one to the content of second counter $C_2$. The content of counter $C_2$ indicates the number of positive judgment or judgements made at Step S26. Subsequently the control goes to Step S29 to judge whether the content of counter $C_2$ is two (i.e., $C_2=2$). If a negative judgment is made at Step S29, i.e., if the content of counter $C_2$ is $C_2=1$, it cannot readily be concluded that the pressing condition of the PW sensor 20 against the body surface 12 has changed, i.e., the pressing condition may remain stable. Thus, the control returns to Step S3. On the other hand, if a positive judgment is made at Step S29, it can be concluded that the pressing condition of the PW sensor 20 has changed. Thus, the control goes to Step S30 to reset the content of counter $C_2$ to $C_2=0$, and subsequently returns to Step S1 to update the optimum chamber pressure Pa and the optimum PS element 31a, hold the chamber pressure P at the updated optimum pressure Pa, and resume detecting the pressure pulse wave of the subject 16 through the updated optimum PS element 31a.

Meanwhile, if a negative judgment is made at Step S23, the control of the CPU 34 goes to Step S25 to conclude that the pattern of change of the curve MTC from the curve $MTC_s$ corresponds to the second pattern, II, more specifically, one of patterns shown in Figs. 8(a) to 8(h) wherein one of the end portions of the current curve MTC largely changed from the corresponding portion of the reference curve $MTC_s$. This result suggests that the change of the pressure pulse wave represented by the change area S did not result from the change of blood pressure of the subject and that the pressing condition of the PW sensor 20 has changed.

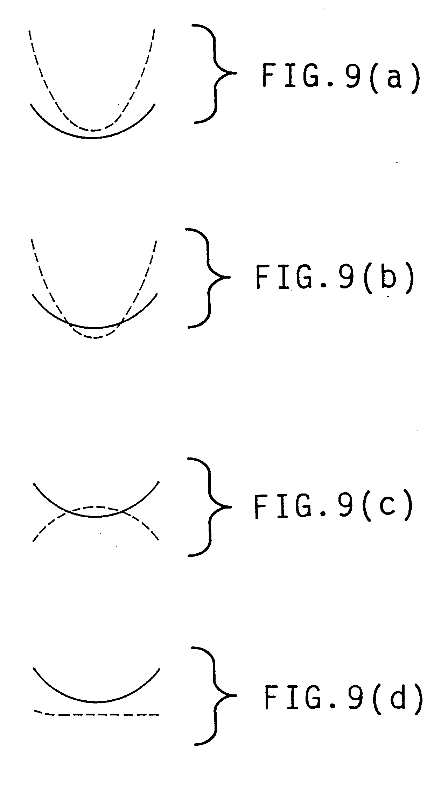

If a negative judgment is made at Step S24, i.e., if the first-end area SL and the second-end area SR are both positive or both negative, the control of the CPU 34 goes to Step S31 to conclude that the pattern of change of the current curve MTC from the reference curve $MTC_s$ corresponds to a third pattern, III, i.e., one of patterns shown in Figs. 9(a) to 9(d) wherein both of the end portions of the current curve MTC largely changed from the corresponding portions of the reference curve $MTC_s$. This result suggests that the change of the pressure pulse wave represented by the change area S did not result from the change of blood pressure of the subject and that the pressing condition of the PW sensor 20 has changed. Step S31 is followed by Step S32 to judge whether the change area S is greater than, e.g., 80. A negative judgment at Step S32 indicates that the pressing condition of the PW sensor 20 has not changed, i.e., remains stable. Therefore, the control of the CPU 34 goes to Step S33 to reset the content of first counter $C_1$ to $C_1=0$, and subsequently returns to Step S3 to continue to detect the pressure pulse wave of the subject 16.

On the other hand, if a positive judgment is made at Step S32, the control of the CPU 34 goes to Step S34 to add one to the content of first counter $C_1$, and subsequently to Step S35 to judge whether the content of counter $C_1$ is two (i.e., $C_1=2$). The counter $C_1$ counts the number of positive judgment or judgments made at Step S32. If a negative judgment is made at Step S35, i.e., if the content of counter $C_1$ is $C_1=1$, it cannot readily be concluded that the pressing condition of the PW sensor 20 against the body surface 12 has changed, i.e., it may remain stable. Thus, the control returns to Step S3. On the other hand, if a positive judgment is made at S35, it can be concluded that the pressing condition of the PW sensor 20 against the body surface 12 has changed. Thus, the control goes to Step S36 to reset the content of first counter $C_1$ to $C_1=0$ and

subsequently returns to Step S1 and following steps to update the optimum chamber pressure Pa and the optimum PS element 31a, hold the chamber pressure P at the updated optimum pressure Pa, and resume detecting the pressure pulse wave of the subject 16 through the updated optimum PS element 31a. In the present embodiment, Steps S14-S36 and a portion of the control device 32 to carry out those steps cooperate with each other to serve as judging means for judging whether the pressing condition of the PW sensor 20 on the body surface 12 is stable, based on the change amount S of the current curve MTC from the reference curve $MTC_s$.

As is apparent from the foregoing description, after the beginning of the pulse wave detection with the PW sensor 20 (i.e., optimum PS element 31a) pressed at the optimum pressing force Pa, the present apparatus periodically judges whether the pressing condition of the PW sensor 20 against the body surface 12 remains stable, based on the amount of change S and/or pattern of change SL, SM, SR of each subsequent curve MTC from the reference curve $MTC_s$ obtained under the stable pressing condition of the PW sensor 20 immediately after the PW sensor 20 is held at the optimum pressure Pa. If a negative judgment is made, the apparatus re-starts the pulse wave detection after re-determining the optimum pressing force Pa. Thus, the pressing condition of the PW sensor 20 with respect to the body surface 12 is adjusted, and therefore the apparatus continuously detects the pressure pulse wave with high accuracy.

While the present invention has been described in its preferred embodiment, the invention may otherwise be embodied.

While in the illustrated embodiment both (a) the change area S representing the change amount of the current curve MTC from the reference curve $MTC_s$ and (b) the three partial areas SL, SM, SR used to identify the change pattern of the curve MTC from the curve $MTC_s$ are utilized for judging whether the pressing condition of the PW sensor 20 against the body surface 12 is stable, it is possible to utilize only one of the two parameters (a) and (b) for the same purpose.

Although in the illustrated embodiment the current curve MTC is translated so that the respective values of the current and reference curves MTC, $MTC_s$ with respect to the optimum PS element 31a coincide with each other, it is possible to translate the curve MTC so that the respective values of the two curves MTC, $MTC_s$ with respect to a predetermined reference PS element 31 (e.g., a middle one in the array) coincide with each other in the coordinate system of Fig. 5.

While in the illustrated embodiment a curve MTC determined immediately after the chamber pressure P is held at the optimum pressure Pa is used as the reference curve $MTC_s$, it is possible to use as the reference curve $MTC_s$ a preceding curve MTC determined in a control cycle preceding a current control cycle where a current curve MTC is determined. In this case, too, the apparatus judges whether the pressing condition of the PW sensor 20 is stable, based on change of the current curve MTC from the reference curve $MTC_s$, i.e., preceding curve MTC.

Although in the illustrated embodiment the apparatus re-starts with Step S1 of Fig. 3(a) when it judges that the pressing condition of the PW sensor 20 is not stable, it is alternatively possible to command the display/record device 40 to display an indication that the pressing condition of the PW sensor 20 has changed, and thereafter terminate the current pulse wave detecting operation. The apparatus may be provided with an alarm device which issues an alarm sound in the same event.

In the illustrated embodiment, Steps S21, S25, and S31 may be omitted from the flow chart of Figs. 4(a) and 4(b).

While in the illustrated embodiment a curve MTC is iteratively determined at regular intervals of 15 seconds based on an average value of respective minimum magnitudes of eight pulses of the pulse wave signal SM from each of the PS elements 31, it is alternatively possible to iteratively determine a curve MTC based on a minimum magnitude of a single pulse of the signal SM from each PS element 31, either at regular intervals of time or for every pulse of the signal SM (i.e., pressure pulse wave of the subject 16).

The illustrated pulse wave detecting apparatus may further incorporate a displacing device as disclosed in U.S. Patent No. 4,901,733. The displacing device includes a motor and a feed screw, and is advantageously used for positioning the PW sensor 20 in a direction intersecting the direction of extension of the radial artery 30. When the apparatus judges that the pressing condition of the PW sensor 20 is not stable, the apparatus may re-position, by operating the displacing device, the PW sensor 20 relative to the radial artery 30 before re-starting with Step S1 of Fig. 3(a).

While in the illustrated embodiment the display/record device 40 displays and records the pressure pulse wave detected through the optimum PS element 31a, the apparatus may further incorporate a blood pressure measuring device including an inflatable cuff for measuring an actual blood pressure value or values of the subject. In this case, the apparatus may be provided with means for determining, in place of or in addition to the pulse-wave display and record functions, a relationship between blood pressure (BP) and pulse-wave magnitude (PW) based on (a) the measured BP value or values and (b) the PW magnitude or magnitudes detected through the PW sensor 20 (i.e., optimum PS element 31a) and continuously determine the BP value or values of the subject 16 for each pulse of the pulse wave of the subject 16 according to the determined BP-PW relationship. This process is described in detail in, for example, U.S. Patent No. 5,099,853.

It is to be understood that the present invention may be embodied with other changes, modifications, and improvements that may occur to those skilled in the art without departing from the scope of the invention defined in the appended claims.

**Claims**

1. A pulse wave detecting apparatus for detecting a pulse wave from a living subject, the pulse wave comprising a plurality of pulses produced from an arterial vessel (30) of the subject in synchronism with heartbeat of the subject, comprising:

   a pulse wave sensor (20) having a press surface (28) and including at least one array of pressure sensing elements (31) provided in said press surface, said press surface of said pulse wave sensor being adapted to be pressed against said arterial vessel of said living subject via a body surface (12) of said subject such that a direction of said array of pressure sensing elements intersects a direction of extension of said arterial vessel, so that each of said pressure sensing elements detects said pulse wave produced from said arterial vessel and generates a pulse wave signal (SM) representing the detected pulse wave;

   a pressing device (18, 22, 24, 26) which produces a pressing force to press said press surface of said pulse wave sensor against said arterial vessel via said body surface; and

   regulating means (32, S1, S2) for changing said pressing force of said pressing device applied to said pulse wave sensor, determining an optimum value of said pressing force based on at least one of the pulse wave signals generated from said pressure sensing elements, and holding said pressing force of said pressing device at the thus determined optimum value;

   the pulse wave detecting apparatus being characterized by comprising:

   minimum magnitude distribution determining means (32, S7-S9) for determining a minimum magnitude of at least one pulse of each of the pulse waves represented by the respective pulse wave signals from said pressure sensing elements, said minimum magnitude distribution determining means iteratively determining a distribution of the respective minimum magnitudes of said pulse waves with respect to said array of pressure sensing elements after said pressing force of said pressing device is held at said optimum value; and

judging means (32, S16, S18, S20, S22-S24, S26, S29, S32, S35) for judging whether a pressing condition of said pulse wave sensor on said body surface is stable, based on changes of the minimum magnitude distributions determined by said minimum magnitude distribution determining means.

2. An apparatus according to claim 1, further comprising control means (32, S19, S30) for controlling, when said judging means (32, S16, S18, S20, S22-S24, S26, S29, S32, S35) provides a negative judgment that said pressing condition of said pulse wave sensor is not stable, said regulating means (32, S1, S2) to update said optimum value of said pressing force and hold said pressing force at the thus updated optimum value.

3. An apparatus according to claim 1 or claim 2, wherein said regulating means (32, S1) determines, by changing said pressing force of said pressing device (18, 22, 24, 26), an amplitude of each of pulses of said at least one of said pulse wave signals (SM) from said pressure sensing elements (31), selects a maximum pulse having a maximum amplitude from said pulses of said at least one pulse wave signal, and determines as said optimum value of said pressing force a pressing force of said pressing device at a time of detection of said maximum pulse.

4. An apparatus according to any of claims 1-3, wherein said regulating means (32, S1) determines, while changing said pressing force of said pressing device (18, 22, 24, 26), an amplitude of each of pulses of each of said pulse wave signals (SM) from said pressure sensing elements (31), selects a maximum pulse having a maximum amplitude from said pulses of said each pulse wave signal, and determines as an optimum pressure sensing element (31a) one of said pressure sensing elements which has detected said maximum pulse.

5. An apparatus according to claim 4, further comprising an output device (40) comprising at least one of (a) a display (40) which displays a waveform of the pulse wave represented by the pulse wave signal (SM) supplied from said optimum pressure sensing element (31a) and (b) a recorder (40) which records a waveform of the pulse wave represented by the pulse wave signal supplied from said optimum pressure sensing element.

6. An apparatus according to any of claims 1-5, wherein said regulating means (32, S2) holds said pressing force of said pressing device (18, 22, 24, 26), at said optimum value, so that said press surface (28) of said pulse wave sensor (20) partially flattens a wall of said arterial vessel (30) and said pressure sensing elements (31) detect said pulse wave through the flattened wall of said arterial vessel.

7. An apparatus according to any of claims 1-6, wherein said minimum magnitude distribution determining means (32, S7-S9) determines a minimum magnitude of each of a predetermined number of pulses of each of said pulse wave signals (SM) from said pressure sensing elements (31) in each of determination cycles, said minimum magnitude distribution determining means determining an average of said predetermined number of minimum magnitudes of said each pulse wave signal and determining a distribution of the respective averages of said pulse wave signals with respect to said array of pressure sensing elements (31), in said each cycle.

8. An apparatus according to any of claims 1-7, wherein said minimum magnitude distribution determining means (32, S9, S11, S12) comprises:

  means (S9, S11) for determining, as a reference distribution, a distribution of respective minimum magnitudes of said pulse waves with respect to said array of pressure sensing elements (31) in one of determination cycles; and

  means (S9, S12) for determining, as a current distribution, a distribution of respective minimum magnitudes of said pulse waves with respect to said array of pressure sensing elements in a current one of said cycles subsequent to said one cycle.

9. An apparatus according to claim 8, wherein said judging means (32, S15-S17) comprises:

  first means (S15) for determining a difference between a minimum magnitude of at least one pulse of the pulse wave from one (31a) of said pressure sensing elements (31) determined in said one cycle and a minimum magnitude of at least one pulse of the pulse wave from said one pressure sensing element determined in said current cycle, obtaining a new minimum magnitude by subtracting said difference from each of the respective minimum magnitudes of said pulse waves from said pressure sensing elements determined in said current cycle, and obtaining an overall amount of change of said current distribution from said reference distribution by summing respective values obtained by subtracting the respective new minimum magnitudes from corresponding minimum magnitudes of said pulse waves from said pressure sensing elements determined in said one cycle;

  second means (S16) for comparing said overall amount of change with a first reference value; and

  third means (S17) for judging that said pressing condition of said pulse wave sensor (20) is stable, when said overall amount of change is smaller than said first reference value.

10. An apparatus according to claim 9, wherein said judging means (32, S18, S19) further comprises:

  fourth means (S18) for comparing said overall amount of change with a second reference value greater than said first reference value; and

  fifth means (S19) for judging that said pressing condition of said pulse wave sensor (20) is not stable, when said overall amount of change is greater than said second reference value.

11. An apparatus according to any of claims 8-10, wherein said judging means (32, S14, S20, S22-S24, S27, S29, S30, S33, S35, S36) comprises:

  first means (S14) for determining a difference between a minimum magnitude of at least one pulse of the pulse wave from each of a first group of pressure sensing elements (31) determined in said one cycle and a minimum magnitude of at least one pulse of the pulse wave from said each pressure sensing element of said first group determined in said current cycle, and obtaining a first amount of change (SL) by summing the respective differences determined for said pressure sensing elements of said first group;

  second means (S14) for determining a difference between a minimum magnitude of at least one pulse of the pulse wave from each of a second group of pressure sensing elements (31) determined in said one cycle and a minimum magnitude of at least one pulse of the pulse wave from said each pressure sensing element of said second group determined in said current cycle, and obtaining a second amount of change (SM) by summing the respective differences determined for said pressure sensing elements of said second group;

  third means (S14) for determining a difference between a minimum magnitude of at least one pulse of the pulse wave from each of a third group of pressure sensing elements (31) determined in said one cycle and a minimum magnitude of at least one pulse of the pulse wave from said each pressure sensing element of said third group obtained in said current cycle, and obtaining a third amount of change (SR) by summing the respective differences determined for said pressure sensing elements of said third group;

said array of pressure sensing elements (31) including said first, second, and third groups of pressure sensing elements such that said first, second, and third groups do not overlap each other in said array;

fourth means (S20, S22-S24) for comparing said first, second, and third amounts of change with each other; and

fifth means (S27, S29, S30, S33, S35, S36) for judging whether said pressing condition of said pulse wave sensor (20) is stable, based on the comparison results obtained by said fourth means.

## Patentansprüche

1. Pulswellenerfassungsvorrichtung zum Erfassen einer Pulswelle von einem Lebewesen, wobei die Pulswelle eine Mehrzahl von Pulsen aufweist, die von einem Arteriengefäß des Wesens synchron zu einem Herzschlag des Wesens erzeugt werden, die aufweist:

   einen Pulswellensensor (20), der eine Druckoberfläche (28) aufweist und mindestens eine Gruppe von druckerfassenden Elementen (31) beinhaltet, die in der Druckoberfläche vorgesehen sind, wobei die Druckoberfläche des Druckwellensensors dazu ausgelegt ist, derart über eine Körperoberfläche (12) des Wesens gegen das Arteriengefäß des Lebewesens gepreßt zu werden, daß eine Richtung der Gruppe von druckerfassenden Elementen eine Ausdehnungsrichtung des Arteriengefäßes kreuzt, so daß jedes druckerfassende Element eine von dem Arteriengefäß erzeugte Druckwelle erfaßt und ein die erfaßte Pulswelle darstellendes Pulswellensignal (SM) erzeugt;

   eine Druckausübungsvorrichtung (18, 22, 24, 26), welche eine Druckkraft erzeugt, um die Druckoberfläche des Druckwellensensors über die Körperoberfläche gegen das Arteriengefäß zu drücken; und

eine Regeleinrichtung (32, S1, S2) zum Ändern der an dem Pulswellensensor ausgeübten Druckkraft der Druckausübungsvorrichtung, zum Bestimmen eines Optimalwerts der Druckkraft auf der Grundlage von mindestens einem der von den druckerfassenden Elementen erzeugten Pulswellensignale und zum Halten der Druckkraft der Druckausübungsvorrichtung an dem derart bestimmten Optimalwert;

wobei die Druckwellenerfassungsvorrichtung dadurch gekennzeichnet ist, daß sie aufweist:

eine Minimalamplitudenverteilungs-Bestimmungseinrichtung (32, S7 bis S9) zum Bestimmen einer Minimalamplitude von mindestens einem Puls von jeder Pulswelle, die durch die jeweiligen Pulswellensignale von den druckerfassenden Elementen dargestellt werden, wobei die Minimalamplitudenverteilungs-Bestimmungseinrichtung iterativ eine Verteilung der jeweiligen Minimalamplituden der Pulswellen bezüglich der Gruppe von druckerfassenden Elementen bestimmt, nachdem die Druckkraft der Druckausübungsvorrichtung an dem Optimalwert gehalten wird; und

eine Entscheidungseinrichtung (32, S16, S18, S22 bis S24, S26, S29, S32, S35) zum Entscheiden, ob ein Druckzustand des Pulswellensensors auf der Körperoberfläche stabil ist, auf der Grundlage von Änderungen der Minimalamplitudenverteilungen, die von der Minimalamplitudenverteilungs-Bestimmungseinrichtung bestimmt werden.

2. Vorrichtung nach Anspruch 1, die weiterhin ein Steuereinrichtung (32, S19, S30) zum Steuern der Regeleinrichtung (32, S1, S2) aufweist, um den Optimalwert der Druckkraft zu aktualisieren und die Druckkraft an dem derart aktualisierten Optimalwert zu halten, wenn die Entscheidungseinrichtung (32, S16, S18, S20, S22 bis S24, S26, S29, S32, S35) eine negative Entscheidung liefert, daß der Druckzustand des Pulswellensensors nicht stabil ist.

3. Vorrichtung nach Anspruch 1 oder 2, bei der die Regeleinrichtung (32, S1) durch Ändern der Druckkraft der Druckausübungsvorrichtung (18, 22, 24, 26) eine Amplitude von jedem Puls des mindestens einen der Pulswellensignale (SM) von den druckerfassenden Elementen (31) bestimmt, einen eine Maximalamplitude aufweisenden Maximalpuls aus den Pulsen des mindestens einen Pulswellensignals auswählt und eine Druckkraft der Druckausübungsvorrichtung zu einer Zeit eines Erfassens des Maximalpulses als den Optimalwert bestimmt.

4. Vorrichtung nach einem der Ansprüche 1 bis 3, bei der die Regeleinrichtung (32, S1) eine Amplitude von jedem Puls von jedem Druckwellensignal (SM) von den druckerfassenden Elementen (31) bestimmt, während sie die Druckkraft der Druckausübungsvorrichtung (18, 22, 24, 26) ändert, einen eine Maximalamplitude aufweisenden Maximalpuls aus den Pulsen von jedem Druckwellensignal auswählt und eines der druckerfassenden Elemente, welches den Maximalpuls erfaßt hat, als ein optimaldruckerfassendes Element (31a) bestimmt.

5. Vorrichtung nach Anspruch 4, die weiterhin eine Ausgabevorrichtung (40) aufweist, die mindestens entweder (a) eine Anzeige (40), welche eine Wellenform der Pulswelle anzeigt, die durch das Pulswellensignal (SM) dargestellt wird, das von dem optimaldruckerfassenden Element (31) geliefert wird, oder (b) eine Aufzeichungsvorrichtung (40) aufweist, welche eine Wellenform der Pulswelle aufzeichnet, die durch das Pulswellensignal dargestellt wird, das von dem optiamldruckerfassenden Element geliefert wird.

6. Vorrichtung nach einem der Ansprüche 1 bis 5, bei der die Regeleinrichtung (32, S2) die Druckkraft der Druckausübungsvorrichtung (18, 22, 24, 26) derart an dem Optimalwert hält, daß die Druckoberfläche (28) des Pulswellensensors (20) teilweise eine Wand des Arteriengefäßes (30) abflacht, und die druckerfassenden Elemente (31) die Pulswelle durch die abgeflachte Wand des Arteriengefäßes erfassen.

7. Vorrichtung nach einem der Ansprüche 1 bis 6, bei der die Minimalamplitudenverteilungs-Bestimmungseinrichtung (32, S7 bis S9) eine Minimalamplitude von jedem einer vorbestimmten Anzahl von Pulsen von jedem Pulswellensignal (SM) von den druckerfassenden Elementen (31) in jedem Bestimmungsyklus bestimmt, wobei die Minimalamplitudenverteilungs-Bestimmungseinrichtung einen Mittelwert der vorbestimmten Anzahl von Minimalamplituden von jedem Pulswellensignal bestimmt und eine Verteilung der jeweiligen Mittelwerte der Pulswellensignale bezüglich der Gruppe von druckerfassenden Elementen (31) in jedem Zyklus bestimmt.

8. Vorrichtung nach einem der Ansprüche 1 bis 7, bei der die Minimalamplitudenverteilungs-Bestimmungseinrichtung (32, S9, S11, S12) aufweist:

eine Einrichtung (S9, S11) zum Bestimmen einer Verteilung von jeweiligen Minimalamplituden der Pulswellen bezüglich der Gruppe von druckerfassenden Elementen (31) in einem Bestimmungszyklus als eine Referenzverteilung; und

eine Einrichtung (S9, S12) zum Bestimmen einer Verteilung von jeweiligen Minimalamplituden der Pulswellen bezüglich der Gruppe von druckerfassenden Elementen in einem gegenwärtigen Zyklus, der dem einen Zyklus nachfolgt, als eine gegenwärtige Verteilung.

9. Vorrichtung nach Anspruch 8, bei der die Entscheidungseinrichtung (32, S15 bis S17) aufweist:

eine erste Einrichtung (S15) zum Bestimmen einer Differenz zwischen einer Minimalamplitude von mindestens einem Puls der Pulswelle von einem (31a) der druckerfassenden Elemente (31), die in dem einen Zyklus bestimmt wird, und einer Minimalamplitude von mindestens einem Puls der Pulswelle von dem einen druckerfassenden Element, die in dem gegenwärtigen Zyklus bestimmt wird, zum Erzielen einen neuen Minimalamplitude durch Subtrahieren der Differenz von jeder der jeweiligen Minimalamplituden der Pulswellen von den druckerfassenden Elementen, die in dem gegenwärtigen Zyklus bestimmt werden, und zum Erzielen eines Gesamthöhe einer Änderung der gegenwärtigen Verteilung aus der Referenzverteilung durch Summieren jeweiliger Werte, die durch Subtrahieren der jeweiligen neuen Minimalamplituden von entsprechenden Minimalamplituden der Pulswellen von den druckerfassenden Elementen erzielt werden, die in dem einen Zyklus bestimmt werden;

eine zweite Einrichtung (S16) zum Vergleichen der Gesamthöhe der Änderung mit einem ersten Referenzwert; und

eine dritte Einrichtung (S17) zum Entscheiden, daß der Druckzustand des Pulswellensensors (20) stabil ist, wenn die Gesamthöhe der Änderung kleiner als der erste Referenzwert ist.

10. Vorrichtung nach Anspruch 9, bei der die Entscheidungseinrichtung (32, S18, S19) weiterhin aufweist:

eine vierte Einrichtung (S18) zum Vergleichen der Gesamthöhe der Änderung mit einem zweiten Referenzwert, der größer als der erste Referenzwert ist; und
eine fünfte Einrichtung zum Entscheiden, daß der Druckzustand des Pulswellensensors (20) nicht stabil ist, wenn die Gesamthöhe der Änderung größer als der zweite Referenzwert ist.

11. Vorrichtung nach einem der Ansprüche 8 bis 10, bei der die Entscheidungseinrichtung (32, S14, S20, S22 bis S24, S27, S29, S30, S33, S35, S36) aufweist:

eine erste Einrichtung (S14) zum Bestimmen einer Differenz zwischen einer Minimalamplitude von mindestens einem Puls der Pulswelle von jedem einer ersten Gruppe von druckerfassenden Elementen (31), die in dem einen Zyklus bestimmt wird, und einer Minimalamplitude von mindestens einem Puls der Pulswelle von jedem druckerfassenden Element der ersten Gruppe, die in dem gegenwärtigen Zyklus bestimmt wird, und zum Erzielen einer ersten Höhe einer Änderung (SL) durch Summieren der jeweiligen Differenzen, die für die druckerfassenden Elemente der ersten Gruppe bestimmt werden;

eine zweite Einrichtung (S14) zum Bestimmen einer Differenz zwischen einer Minimalamplitude von mindestens einem Puls der Pulswelle von jedem einer zweiten Gruppe von druckerfassenden Elementen (31), die in dem einen Zyklus bestimmt wird, und einer Minalamplitude von mindestens einem Puls der Pulswelle von jedem druckerfassenden Element der zweiten Gruppe, die in dem gegenwärtigen Zyklus bestimmt wird, und zum Erzielen einer zweiten Höhe einer Änderung (SM) durch Summieren der jeweiligen Differenzen, die für die druckerfassenden Elemente der zweiten Gruppe bestimmt werden;

eine dritte Einrichtung (S14) zum Bestimmen einer Differenz zwischen einer Minimalamplitude von mindestens einem Puls der Pulswelle von jedem einer dritten Gruppe von druckerfassenden Elementen (31), die in dem einen Zyklus bestimmt werden, und einer Minimalamplitude von mindestens einem Puls der Pulswelle von jedem druckerfassenden Element der dritten Gruppe, die in dem gegenwärtigen Zyklus erzielt wird, und zum Erzielen einer dritten Höhe einer Änderung (SR) durch Summieren der jeweiligen Differenzen, die für die druckerfassenden Elemente der dritten Gruppe bestimmt werden;
wobei die Gruppe von druckerfassenden Elementen (31) die ersten, zweiten und dritten Gruppen von druckerfassenden Elementen derart beinhaltet, daß die ersten, zweiten und dritten Gruppen einander in der Gruppe nicht überlappen;

eine vierte Einrichtung (S20, S22 bis S24) zum miteinander Vergleichen der ersten, zweiten und dritten Höhen einer Änderung; und

eine fünfte Einrichtung (S27, S29, S30, S33, S35, S36) zum Entscheiden, ob der Druckzustand des Pulswellensensors (20) stabil ist, auf der Grundlage der Vergleichsergebnisse, die von der vierten Einrichtung erzielt werden.

## Revendications

1. Appareil de détection d'onde d'impulsion destiné à détecter une onde d'impulsion issue d'un sujet vivant, l'onde d'impulsion comprenant une pluralité d'impulsions produites à partir d'un vaisseau artériel (30) du sujet en synchronisme avec les battements de coeur du sujet, comprenant :

un capteur (20) d'onde d'impulsion comportant une surface (28) de pression et comprenant au moins un groupement d'éléments (31) de détection de pression disposés dans ladite surface de pression, ladite surface de pression dudit capteur d'onde d'impulsion étant susceptible d'être pressée contre ledit vaisseau artériel dudit sujet vivant via une surface corporelle (12) dudit sujet de façon qu'une direction dudit groupement d'éléments de détection de pression coupe une direction d'étendue dudit vaisseau artériel, de façon que chacun desdits éléments de détection de pression détecte ladite, onde d'impulsion produite à partir du vaisseau artériel et crée un signal (SM) d'onde d'impulsion représentant l'onde d'impulsion détectée ;
un dispositif (18, 22, 24, 26) de pression qui produit une force de pression pour presser ladite surface de pression dudit capteur d'onde d'impulsion contre ledit vaisseau artériel via ladite surface corporelle ; et
un moyen (32, S1, S2) de régulation destiné à faire varier ladite force de pression dudit dispositif de pression appliquée audit capteur d'onde d'impulsion, en déterminant une valeur optimale de ladite force de pression basée sur au moins l'un des signaux d'onde d'impulsion produits à partir desdits éléments de détection de pression, et en maintenant ladite force de pression dudit dispositif de pression à la valeur optimale ainsi déterminée ;
l'appareil de détection d'onde d'impulsion se caractérisant en ce qu'il comprend :
un moyen (32, S7 à S9) de détermination de répartition d'amplitude minimale destiné à déterminer une amplitude minimale d'au moins une impulsion de chacune des ondes d'impulsion représentées par les signaux d'onde d'impulsion respectifs provenant desdits éléments de détection de pression, ledit moyen de détermination de répartition d'amplitude minimale déterminant de façon répétée une répartition des amplitudes minimales respectives desdites ondes d'impulsion par rapport audit groupement d'éléments de détection de pression, dès que ladite force de pression dudit dispositif de pression est maintenue à ladite valeur optimale ; et

un moyen (32, S16, S18, S20, S22 à S24, S26, S29, S32, S35) de détermination destiné à déterminer si un état de pression dudit capteur d'onde d'impulsion sur ladite surface corporelle est stable, basé sur des variations des répartitions d'amplitude minimale déterminées par ledit moyen de détermination de répartition d'amplitude minimale.

2. Appareil selon la revendication 1, comprenant en outre un moyen (32, S19, S30) de commande destiné à commander, lorsque ledit moyen (32, S16, S18, S20, S22 à S24, S26, S29, S32, S35) de détermination fournit une détermination négative indiquant que ledit état de pression dudit capteur d'onde d'impulsion n'est pas stable, ledit moyen (32, S1, S2) de régulation pour mettre à jour ladite valeur optimale de ladite force de pression et maintenir ladite force de pression à la valeur optimale ainsi mise à jour.

3. Appareil selon la revendication 1, ou la revendication 2, dans lequel ledit moyen (32, S1) de régulation détermine, en faisant varier ladite force de pression dudit dispositif (18, 22, 24, 26) de pression, une amplitude de chacune des impulsions dudit au moins un desdits signaux (SM) d'onde d'impulsion provenant desdits éléments (31) de détection de pression, choisit une impulsion maximale ayant une amplitude maximale parmi lesdites impulsions dudit au moins un signal d'onde d'impulsion, et détermine, en tant que dite valeur optimale de ladite force de pression, une force de pression dudit dispositif de pression à un instant de détection de ladite impulsion maximale.

4. Appareil selon l'une quelconque des revendications 1 à 3, dans lequel ledit moyen (32, S1) de régulation détermine, tout en faisant varier ladite force de pression dudit dispositif (18, 22, 24, 26) de pression, une amplitude de chacune des impulsions de chacun desdits signaux (SM) d'onde d'impulsion provenant desdits éléments (31) de détection de pression, choisit une impulsion maximale ayant une amplitude maximale parmi lesdites impulsions de chaque signal d'onde d'impulsion, et détermine, en tant qu'élément (31a) de détection de pression optimale l'un desdits éléments de détection de pression qui a détecté ladite impulsion maximale.

5. Appareil selon la revendication 4, comprenant en outre un dispositif (40) de sortie comprenant au moins l'un (a) d'un afficheur (40) qui affiche une forme d'onde de l'onde d'impulsion représentée par le signal (SM) d'onde d'impulsion délivré par ledit élément (31a) de détection de pression optimale, et (b) d'un enregistreur (40) qui enregistre une forme d'onde de l'onde d'impulsion représentée par le signal d'onde d'impulsion délivré par ledit élément de détection de pression optimale.

6. Appareil selon l'une quelconque des revendications 1 à 5, dans lequel ledit moyen (32, S2) de régulation maintient ladite force de pression dudit dispositif (18, 22, 24, 26) de pression, à ladite valeur optimale, de façon que ladite surface (28) de pression dudit capteur (20) d'onde d'impulsion aplatisse partiellement une paroi dudit vaisseau artériel (30) et que lesdits éléments (31) de détection de pression détectent ladite onde d'impulsion à travers la paroi aplatie dudit vaisseau artériel.

7. Appareil selon l'une quelconque des revendications 1 à 6, dans lequel ledit moyen (32, S7 à S9) de détermination de répartition d'amplitude minimale détermine une amplitude minimale de chacune d'un nombre prédéterminé d'impulsions de chacun desdits signaux (SM) d'onde d'impulsion provenant desdits éléments (31) de détection de pression pendant chacun des cycles de détermination, ledit moyen de détermination de répartition d'amplitude minimale déterminant une moyenne dudit nombre prédéterminé d'amplitudes minimales de chaque signal d'onde d'impulsion et déterminant une répartition des moyennes respectives desdits signaux d'onde d'impulsion par rapport audit groupement d'éléments (31) de détection de pression, pendant chaque cycle.

8. Appareil selon l'une quelconque des revendications 1 à 7, dans lequel ledit moyen (32, S9, S11, S12) de détermination de répartition d'amplitude minimale comprend :

un moyen (S9, S11) destiné à déterminer, en tant que répartition de référence, une répartition d'amplitudes minimales respectives desdites ondes d'impulsion par rapport audit groupement d'éléments (31) de détection de pression pendant l'un des cycles de détermination ; et

un moyen (S9, S12) destiné à déterminer, en tant que répartition de courant, une répartition d'amplitudes minimales respectives desdites ondes d'impulsion par rapport audit groupement d'éléments de détection de pression pendant l'un, en cours, desdits cycles suivant ledit un cycle.

9. Appareil selon la revendication 8, dans lequel ledit moyen (32, S15 à S17) de détermination comprend :

un premier moyen (S15) destiné à déterminer une différence entre une amplitude minimale d'au moins une impulsion de l'onde d'impulsion provenant de l'un (31a) desdits éléments (31) de détection de pression déterminé pendant ledit un cycle et une amplitude minimale d'au moins une impulsion de l'onde d'impulsion provenant dudit un élément de détection de pression déterminé pendant ledit cycle en cours, en obtenant une nouvelle amplitude minimale en soustrayant ladite différence de chacune des amplitudes minimales respectives desdites ondes d'impulsion provenant desdits éléments de détection de pression déterminés pendant ledit cycle en cours, et en obtenant une valeur de variation totale de ladite répartition de courant à partir de ladite répartition de référence en sommant des valeurs respectives obtenues en soustrayant les nouvelles amplitudes minimales respectives des amplitudes minimales correspondantes desdites ondes d'impulsion provenant desdits éléments de détection de pression déterminés pendant ledit un cycle ;
un deuxième moyen (S16) destiné à comparer ladite valeur de variation totale avec une première valeur de référence ; et
un troisième moyen (S17) destiné à déterminer que ledit état de pression dudit capteur (20) d'onde d'impulsion est stable, lorsque ladite valeur de variation totale est plus petite que ladite première valeur de référence.

10. Appareil selon la revendication 9, dans lequel ledit moyen (32, S18, S19) de détermination comprend en outre :

un quatrième moyen (S18) destiné à comparer ladite valeur de variation totale avec une seconde valeur de référence plus grande que ladite première valeur de référence ; et
un cinquième moyen (S19) destiné a déterminer que ledit état de pression dudit capteur (20) d'onde d'impulsion n'est pas stable, lorsque ladite valeur de variation totale est plus grande que ladite seconde valeur de référence.

11. Appareil selon l'une quelconque des revendications 8 à 10, dans lequel ledit moyen (32, S14, S20, S22 à S24, S27, S29, S30, S33, S35, S36) de détermination comprend :

un premier moyen (S14) destiné à déterminer une différence entre une amplitude minimale d'au moins une impulsion de l'onde d'impulsion provenant de chacun d'un premier groupe d'éléments (31) de détection de pression déterminé pendant ledit un cycle et une amplitude minimale d'au moins une impulsion de l'onde d'impulsion provenant de chaque élément de détection de pression dudit premier groupe déterminé pendant ledit cycle en cours, et en obtenant une première valeur de variation (SL) en sommant les différences respectives déterminées pour lesdits éléments de détection de pression dudit premier groupe ;
un deuxième moyen (S14) destiné à déterminer une différence entre une amplitude minimale d'au moins une impulsion de l'onde d'impulsion provenant de chacun d'un deuxième groupe d'éléments (31) de détection de pression déterminé pendant ledit un cycle et une amplitude minimale d'au moins une impulsion de l'onde d'impulsion provenant de chaque élément de détection de pression dudit deuxième groupe, déterminé pendant ledit cycle en cours, et en obtenant une deuxième valeur de variation (SM) en sommant les différences respectives déterminées pour lesdits éléments de détection de pression dudit deuxième groupe ;
un troisième moyen (S14) destiné à déterminer une différence entre une amplitude minimale d'au moins une impulsion de l'onde d'impulsion provenant de chacun d'un troisième groupe d'éléments (31) de détection de pression, déterminé pendant ledit un cycle, et une amplitude minimale d'au moins une impulsion de l'onde d'impulsion provenant de chaque élément de détection de pression dudit troisième groupe, obtenu pendant ledit cycle en cours, et en obtenant une troisième valeur de variation (SR) en sommant les différences respectives déterminées pour lesdits éléments de détection de pression dudit troisième groupe ;
ledit groupement d'éléments (31) de détection de pression comprenant lesdits premier, deuxième et troisième groupes d'éléments de détection de pression de sorte que lesdits premier, deuxième et troisième groupes ne se chevauchent pas mutuellement dans ledit groupement ;
un quatrième moyen (S20, S22 à S24) destiné à comparer lesdites première, deuxième et troisième valeurs de variation les unes avec les autres ; et
un cinquième moyen (S27, S29, S30, S33, S35, S36) destiné à déterminer si ledit état de pression dudit capteur (20) d'onde d'impulsion est stable, sur la base des résultats de comparaison obtenus par ledit quatrième moyen.

FIG.1

FIG.2

EP 0 671 144 B1

# FIG.3(a)

```
        ┌─────────────────────┐
        │       START         │
        └─────────────────────┘
                  │        ◄─────────────  ③
        ┌─────────────────────┐
        │ OPTIMUM PRESSURE Pa &│
        │ OPTIMUM ELEMENT 31a  │
        │ DETERMINED           │  S1
        └─────────────────────┘
                  │
        ┌─────────────────────┐
        │ CHAMBER PRESSURE P   │
        │ HELD AT OPTIMUM      │
        │ PRESSURE Pa          │  S2
        └─────────────────────┘
                  │        ◄─────────────  ②
        ┌─────────────────────┐
        │ ONE PULSE DETERMINED │
        │ AND STORED           │  S3
        └─────────────────────┘
                  │
        ┌─────────────────────┐
        │ ONE PULSE DISPLAYED  │
        │ AND RECORDED         │  S4
        └─────────────────────┘
                  │        ◄─────────────  ④
                  ▼         F = 1 ?
              ◇ S5 ◇ ──────────────────►  ⑤
                  │          YES
                  │ NO
                  ▼        EIGHT PULSES ?
              ◇ S6 ◇ ──────────────────►  ②
                  │          NO
                  │ YES
        ┌─────────────────────┐
        │ LOWER-PEAK MAGNITUDES│
        │ OF EIGHT PULSES      │
        │ DETERMINED FOR EACH  │
        │ ELEMENT 31           │  S7
        └─────────────────────┘
                  │
        ┌─────────────────────┐
        │ AVERAGE OF EIGHT     │
        │ LOWER-PEAK MAGNITUDES│
        │ DETERMINED FOR EACH  │
        │ ELEMENT 31           │  S8
        └─────────────────────┘
                  │
        ┌─────────────────────┐
        │ LOWER-PEAK VARIATION │
        │ DETERMINED           │  S9
        └─────────────────────┘
                  │
                 ⑥
```

18

# FIG.3(b)

⑥

| F ← 1 | S10 |

FIRST LOWER-PEAK
VARIATION ?

S11

~~YES~~
NO ·

~~NO~~

YES

| AREAS SL, SM, SR DETERMINED | S14 |

| AREA S DETERMINED | S15 |

①

⑤

FIFTEEN
SECONDS ?

S12 →NO→ ②

YES

| F ← 0 | S13 |

④

# FIG.4(a)

```
                    (1)
                     │        S ≦ 80 ?
              ┌──────────────┐
              │     S16      ├─────────────────────────────┐
              └──────────────┘   YES              S17       │
                     │ NO                    ┌──────────────────────┐
         YES         │        S > 400 ?      │  C1← 0, C2← 0        │
   S19  ┌──────────────────┐                 └──────────────────────┘
        │      S18         │                          │
┌────────────────┐  └──────┘                        (3)
│  C1← 0, C2← 0  │     │ NO      SL = SM AND
└────────────────┘     │         SM = SR ?
        │       ┌──────────────┐
       (3)      │     S20      │
                └──────────────┘  YES
                     │ NO
                     │        │ SL │ < │ SM │  AND
                     │        │ SM │ > │ SR │  ?
              ┌──────────────┐                    S21
              │     S22      ├──────────────┐  ┌──────────────┐
              └──────────────┘   YES        └─▶│  PATTERN     │
                     │ NO                       │  GROUP I     │
                    (7)                         └──────────────┘
```

EP 0 671 144 B1

# FIG.4(b)

⑦

$|SL| > |SM|$ AND
$|SM| < |SR|$ ?

◇ S23
NO
YES

$SL \cdot SR < 0$ ?
◇ S24
NO
YES

┌─────────────┐
│ PATTERN     │  S25
│ GROUP II    │
└─────────────┘

$S > 150$ ?
◇ S26
NO
YES

┌─────────────┐          S27
│ C2 ← C2 + 1 │  S28    ┌──────────┐
└─────────────┘          │ C2 ← 0   │
                         └──────────┘
$C2 = 2$ ?
◇ S29
NO
YES

┌──────────┐  S30
│ C2 ← 0   │
└──────────┘

②

③

S31
┌─────────────┐
│ PATTERN     │
│ GROUP III   │
└─────────────┘

$S > 80$ ?
◇ S32
NO
YES

┌─────────────┐          S33
│ C1 ← C1 + 1 │  S34    ┌──────────┐
└─────────────┘          │ C1 ← 0   │
                         └──────────┘
$C1 = 2$ ?
◇ S35
NO
YES

┌──────────┐  S36
│ C1 ← 0   │
└──────────┘

③

②

21

EP 0 671 144 B1

# FIG.5

# FIG.6

22

FIG.7(a)

FIG.7(b)

FIG.7(c)

FIG.7(d)

FIG.7(e)

EP 0 671 144 B1

FIG.7(f)

FIG.7(g)

FIG.7(h)

FIG.7(i)

FIG.7(j)

24

FIG.8(a)

FIG.8(b)

FIG.8(c)

FIG.8(d)

FIG.8(e)

FIG.8(f)

FIG.8(g)

FIG.8(h)

FIG.8(i)

FIG.8(j)

FIG.8(k)

FIG.8( ℓ )

FIG.9(a)

FIG.9(b)

FIG.9(c)

FIG.9(d)